(19)

Europäisches Patentamt
European Patent Office
Office européen des brevets

(11) **EP 3 593 706 A1**

(12) **EUROPEAN PATENT APPLICATION**

(43) Date of publication:
**15.01.2020 Bulletin 2020/03**

(51) Int Cl.:
**A61B 5/00** (2006.01)         **A61B 5/021** (2006.01)
**A61B 5/027** (2006.01)

(21) Application number: **18182826.0**

(22) Date of filing: **11.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **FRACKOWIAK, Bruno Jean François**
**5656 AE Eindhoven (NL)**
• **VAN DER HORST, Arjen**
**5656 AE Eindhoven (NL)**
• **SHULEPOV, Sergei**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **PULSE-WAVE VELOCITY DETERMINATION DEVICE, SYSTEM AND METHOD**

(57) The invention is directed to a device (100) for determining a value of a pulse-wave velocity in a blood vessel under measurement, that comprises a first measurement data input unit (102) configured to receive data indicative of vessel geometry parameters of the blood vessel, a second measurement data input unit (104) configured to receive data representing a measured transmission velocity amount of pressure waves in the blood vessel in presence of an elongated device having predetermined spatial extension values and a pulse-wave velocity determination unit (106), which is configured to determine, using the vessel-geometry data and the spatial extension values of the elongated device, a geometry factor dependent on a cross-sectional area of the blood vessel that remains available for blood flow in the presence of the elongated device and to determine and output the value of the pulse-wave velocity using the second measurement data and the geometry factor.

FIG. 1

**Description**

FIELD OF THE INVENTION

The present invention is directed to a device for determining a value of a pulse-wave velocity in a region of a blood vessel, to a system for determining a value of a pulse-wave velocity in a region of a blood vessel, to a method for operating a device for determining a value of a pulse-wave velocity in a region of a blood vessel, to a method for operating a system for determining a value of a pulse-wave velocity in a region of a blood vessel and to a computer program.

BACKGROUND OF THE INVENTION

US 2010/0113949 A1 describes systems and methods that enable measurement of the velocity of a pulse wave propagating within a body lumen such as a blood vessel using a pulse-wave velocity determining device such as an intravascular elongate medical device. The elongate medical device can include a data collection device configured to collect pulse wave data at a location within the lumen. The data collection device is communicatively coupled with a velocity measurement system and configured to output the collected data to the velocity measurement system for determining a value of a pulse-wave velocity. The velocity measurement system is configured to calculate the velocity of the pulse wave based on the collection data. The velocity of a pulse wave over a region of the lumen can be used for tissue characterization, diagnosis and the like.

SUMMARY OF THE INVENTION

It would be beneficial to improve the determination of the pulse wave velocity.
According to a first aspect of the present invention, a device for determining a value of a pulse-wave velocity in a region of a blood vessel under measurement is described. The device comprises:

- a first measurement data input unit configured to receive first measurement data indicative of vessel geometry parameters of the blood vessel;
- a second measurement data input unit configured to receive second measurement data representing a measured transmission velocity amount of pressure or flow waves in the region of the blood vessel in presence of an inserted elongated device which has predetermined spatial extension values.

The device of the first aspect further comprises a pulse-wave-velocity determination unit, which is configured:

- to determine, using the vessel-geometry data and the spatial extension values of the inserted elongated device, a geometry factor dependent on a cross-sectional area of the blood vessel that remains available for blood flow in the presence of the inserted elongated device and to determine and output the value of the pulse-wave-velocity using the second measurement data and the geometry factor.

The inventors have recognized that the significance of the determined value of the pulse wave velocity is improved by taking into account the presence of an inserted elongated device inside the blood vessel under measurement. Typical inserted elongated devices include, but are not limited to, transmission-velocity determining devices for determining transmission velocity of pulse waves in a vessel. Other inserted elongated devices that affect the significance of the pulse wave velocity are catheters and guidewires used for other purposes than determining the transmission velocity, such as ablation catheters. Regardless of the purpose of the inserted elongated device, the pulse wave velocity at the region of the blood vessel under measurement is influenced by its presence and is thus different from the pulse wave velocity expected without the inserted elongated device. The inserted elongated device is an external device that does not form part of the device of the first aspect of the invention.
The inventors have found that the effect of the presence of the inserted elongated device is larger for smaller blood vessels and for larger elongated devices. The geometry factor that is determined by the pulse-wave-velocity determination unit of the device of the present invention is dependent on a cross-sectional area of the blood vessel that remains available for blood flow in the presence of the inserted elongated device.
In the device of the present invention, the first measurement data is received via a first measurement data input unit and is indicative of vessel geometry parameters of the blood vessels. The second measurement data is received at a second measurement data input unit and represent a measured transmission velocity amount of pressure or flow waves in the region of the blood vessel in the presence of an inserted elongated device located at that portion of the blood vessel that is under measurement. In contrast to known prior-art devices for providing a value of the pulse-wave velocity output, the device of the first aspect of the present invention uses the first measurement data and the second measurement data to determine the geometry factor that is then advantageously used to increase the significance of the determined value of the pulse-wave velocity.
The value of the pulse-wave-velocity is determined at the pulse-wave-velocity determination unit using the second measurement data that represents the measured transmission velocity of the pressure or flow waves and the geometry factor. The determined value of the pulse-wave velocity has a higher significance than values provided by known devices, since it accounts for the effect of a presence of the elongated device on the blood flow, and therefore, on the pressure or flow waves at the region of the blood vessel under measurement.

Therefore, the device of the first aspect of the invention provides a more accurate and significant value of the pulse-wave velocity and provides, in a reasonable time, useful insights about blood flow physiology and pulse wave propagation artefacts.

In the following, embodiments of the device of the first aspect of the invention will be described.

In an embodiment, the pulse-wave-velocity determination unit is configured to determine, using the first measurement data, a first extension value indicative of an average radius of the region of the blood vessel. Further, the pulse-wave-velocity determination unit is configured to receive a second extension value indicative of an average radius of a predetermined longitudinal section of the inserted elongated device. The pulse-wave-velocity determination unit is then configured to determine the geometry factor using the first extension value and the second extension value and to output the value of the pulse-wave-velocity using the second measurement data and the geometry factor. In this embodiment, the geometry factor is determined based on the average radius of the region of the blood vessel under measurement and on the average radius of that longitudinal section of the elongated device that is intended to be arranged at the region of the blood vessel. In an alternative embodiment, the, pulse-wave-velocity determination unit is configured to receive the first extension value indicative of the average radius of the region of the blood vessel, for example, as a manual input. Typically, the radius of the predetermined longitudinal section of the elongated device intended to be inserted in the region of the blood vessel under measurement has a constant value within the limits of fabrication. If an elongated device is used that does not have a constant radius along the predetermined longitudinal section, the average radius is advantageously used.

In another embodiment, the pulse-wave-velocity determination unit is configured to use the first and the second extension values to determine the geometry factor as $\sqrt{1 - \dfrac{r_2^2}{r_1^2}}$, wherein $r_1$ is the first extension value indicative of the average radius of the region of the blood vessel and $r_2$ is the second extension value indicative of the average radius of a predetermined longitudinal section of the elongated device. The value of the pulse-wave-velocity is then determined by dividing the measured transmission velocity amount by the geometry factor.

Alternatively, another embodiment of the device for determining a value of a pulse-wave velocity comprises a pulse-wave-velocity determination unit that is configured to determine the geometry factor by performing a simulation based on a computational fluid dynamics (CFD) model using as inputs the vessel geometry parameters of the blood vessel and the spatial extension values of the elongated device. In particular, the simulation is performed using a simplified geometry model based on a cylinder having a tube inside, the tube and the cylinder being arranged concentrically, and wherein a fluid is allowed to flow in a flow volume located between an outer wall of the tube and an inner wall of the cylinder. The flow volume has thus, in this simplified geometry, an annular cross section in a plane perpendicular to a longitudinal direction of the cylinder and a tube. A wall deformation due to the pulse waves is modelled using a fluid structure interaction (FSI) model. Since FSI requires a relatively high computational power, the CFD and the FSI models advantageously used an axisymmetric approach wherein the blood vessel is modelled as a wedge with a symmetry conditions at both longitudinal side planes of the wedge. A list of parameters for the simulation include a blood vessel diameter value or a blood vessel radius value, a blood vessel length value and a vessel wall thickness value that are obtained from the vessel geometry parameters. The list of parameters also includes a transmission-velocity determining device diameter or radius value that are obtained from the spatial extension values of the elongated device.

A value of an elastic modulus of a region of a blood vessel is related to a stiffening of said region. Arterial stiffening is an important risk factor for cardiovascular disease. Arterial stiffness increases with aging and various disease states, including hypertension, hypercholesterolemia, diabetes mellitus, obesity, smoking, and kidney disease. Moreover, for some specific interventions like bypass or stent placement, the vessel must be stiff enough to prevent damage and complications. A commonly used parameter to assess the stiffness of a blood vessel is the pulse wave velocity PWV. PWV is the transmission speed of pressure or flow waves through the blood vessels, for instance generated by the beating heart. The compliant vessel wall acts as a spring, i.e. as a mass system triggered by the pulsed blood flow, and the resulting PWV is determined by the ability of the vessel to expand (i.e. distensibility, $D$) according to the following relation:

$$PWV = \sqrt{\frac{1}{\rho D}} \text{ with } D = \frac{dV}{VdP}$$

wherein $V$ is a vascular volume, $P$ represents the pressure and $\rho$ is the blood density. In this relation, the relative volume variation of flow can be related to the radial displacement of the vessel wall, whereas the pressure variation acts directly as a load to the vessel wall. Hence the Moens-Korteweg equation can be derived:

$$PWV = \sqrt{\frac{E \cdot h}{d \cdot \rho}}$$

Another embodiment of the device of the first aspect of the invention is advantageously configured to further provide, based on the value of the pulse-wave-velocity with an increased significance, a value of an elastic-modulus of the region of the blood vessel under measurement, that also has an increased significance. This embodiment also comprises an elastic-modulus determination unit configured to receive, from the pulse-wave-velocity determination unit, the value of the pulse-wave-velocity and the average radius of the region of the blood vessel. It is further configured to receive, via a data input, a vessel-wall thickness value indicative of a thickness of the wall of the blood vessel, and a blood density value, indicative of the density of the blood circulating inside the blood vessel and to determine the value of the elastic modulus of the blood vessel using the value of the pulse-wave-velocity, the vessel-wall thickness value and the blood density value by determining:

$$E = \frac{PWV^2}{h} \cdot 2r_1 \cdot \rho \ ,$$

wherein

$E$ is the value of the elastic modulus of the blood vessel having an increased significance,
$PWV$ is the value of the pulse-wave-velocity determined by the pulse-wave-velocity determination unit,
$h$ is the vessel thickness value of the blood vessel received via the data input,
$r_1$ is the average radius of the region of the blood vessel, and
$\rho$ is the blood density value, also received via the data input.

The value of the vessel thickness and the value of the blood density can be patient specific or be determined from population data. For instance, the vessel thickness value can be derived from intravascular imaging data provided, for example, by an intravascular ultrasound (IVUS) probe or by optical coherence tomography (OCT). Alternatively, the vessel thickness value can be derived from an external imaging device such as, but not limited to an ultrasound device or a magnetic resonance imaging device. In a simple embodiment, value of the vessel thickness and/or the value of the blood density are received as a manual input.

According to a second aspect of the present invention, a system for determining a value of a pulse-wave velocity in a region of a blood vessel under measurement is described. The system comprises:

- a device for determining a value of a pulse-wave velocity in a region of a vessel under measurement in accordance with the first aspect of the present invention;
- an imaging unit for providing the first measurement data indicative of vessel geometry parameters of the vessel, the imagining unit connected to the first measurement data input unit of the device; and
- a transmission-velocity measurement device for providing the second measurement data representing a measured transmission velocity amount of pressure or flow waves in the region of the vessel, the transmission-velocity measurement device being connected to the second measurement data input unit of the device.

The system of the second aspect thus shares the advantages of the device of the first aspect or any of its embodiments. Thus, any of the embodiments of the device for determining a value of a pulse-wave velocity of the first aspect can be used in a system in accordance with the second aspect.

The system of the second aspect of the present invention, in addition to the device of the first aspect, includes an imaging unit and a transmission-velocity measurement device. The imaging unit is connected to the first measurement data input unit of the device and provides the first measurement data. The transmission-velocity measurement device is connected to the second measurement data input unit and provides the second measurement data.

In the following, embodiments of the system of the second aspect of the invention will be described.

In an embodiment, the imaging unit is further connected to the second measurement data input unit and configured to determine and provide the spatial extension values of the elongated device. This is particularly advantageous in cases where the first measurement data indicative of vessel geometry parameters of the vessel is further indicative of the spatial extension values of the elongated device.

In a particular embodiment of the system of the invention, the imaging unit comprises an image-acquiring device that is configured to provide image data indicative of the region of the blood vessel and a segmentation unit that is configured to receive the image data and to perform a segmentation algorithm on the image data for determining and providing the vessel-geometry data indicative of the vessel geometry.

In alternative embodiments of the system of the second aspect, the image-acquiring device comprises an ultrasound probe or a computed tomography scanner or a magnetic resonance imaging scanner or an angiography-imaging device, particularly an x-ray angiography-imaging device, that is configured to provide the image data indicative of the blood vessel.

The image-acquiring device is configured to provide a detailed 3D image sequence of the blood vessel. A contrast agent may be injected in blood to enhance the vessel shape on the image data. Afterwards, a segmentation software performing a corresponding segmentation algorithm is used to convert the 3D image sequence into a geometrical representation of blood vessels. The vessel geometry parameters are obtained from the reconstructed vessel geometry. For instance, the segmentation unit is configured to provide a blood vessel diameter value in every location along the vessel branch. Hence it is possible to determine the average diameter of the vessel corresponding to the region of the blood vessel where the pulse-wave velocity is intended to be measured.

Another embodiment of the system of the second aspect additionally or alternatively comprises a transmission-velocity measurement device including a catheter or a guidewire having predetermined spatial extension values and configured to be inserted into the blood vessel for determining the transmission velocity. The catheter or the guidewire include at least two pulse wave sensors arranged at respective predetermined positions along a longitudinal direction of the catheter or the guidewire. The pulse wave sensors are configured to detect pulse waves propagating along the blood vessel. A respective time difference $\Delta t$ between detection of pulse waves at the respective pulse wave sensors is determined. Since a distance value between the pulse wave sensors $x$ is also known, the value of the pulse wave velocity is obtained as

$$PWV = \frac{x}{\Delta t} \qquad .$$

In another embodiment, the transmission-velocity measurement device is a device that is not configured to be inserted into the blood vessel, such as, for instance, an ultra-high speed ultrasound probe. This embodiment is suitable for increasing the significance of the measured transmission velocity in cases where there is an elongated device inserted in the region of the blood vessel under measurement and where the elongated device is inserted for a purpose other than measuring the transmission velocity, such as, for instance, a treatment catheter (e.g. ablation catheter).

Regardless of the nature of the inserted elongated device, the value of the pulse wave velocity, which is provided by the transmission-velocity measurement device to the pulse-wave velocity determination unit, is influenced by the presence of the inserted elongated device at the region of the blood vessel under measurement. The significance of the measured pulse wave velocity is enhanced by the device of the first aspect by determining and applying a geometry factor accounting for the effects of the presence of inserted elongated device inside the blood vessel.

In alternative embodiments, the pulse wave sensors arranged on the catheter or guidewire of the transmission-velocity determining device are pressure sensors or flow sensors or imaging sensors or a combination thereof.

In a particular embodiment, the catheter or guidewire comprises a first flow sensor and/or a first pressure sensor at a first position along the longitudinal direction and a second flow sensor and/or a second pressure sensor at a second position along the longitudinal direction, the first and the second positions being arranged at a predetermined distance amount from each other along the longitudinal direction.

In another embodiment, the transmission velocity measurement device comprises two or more catheters or guidewires having predetermined spatial extension values, each catheter or guidewire including at least one pulse wave sensor arranged at respective predetermined positions along a longitudinal direction of the respective catheter or guidewire, and the sensors located at an axial distance from each other.

In this case $r_2^2 = \sum r_{2,i}^2$, where $i$ is the number of catheters or guidewires within the vessel.

In particular, pressure sensors provide a higher accuracy than flow sensors. However, accuracy of flow sensors has been increasing in the last years and they are also suitable for determining the transmission velocity of pulse waves.

According to a third aspect of the present invention, a method for operating a device for determining a value of a pulse-wave velocity in a region of a blood vessel under measurement is described. The method comprises:

- receiving, at a first measurement data input unit, first measurement data indicative of vessel geometry parameters of the blood vessel;
- receiving, at a second measurement data input unit, second measurement data representing a measured transmission velocity amount of pressure or flow waves in the region of the vessel in presence of an inserted elongated device having predetermined spatial extension values;
- determining, using the vessel-geometry data and the spatial extension values of the inserted elongated device, a geometry factor dependent on a cross-sectional area of the blood vessel that remains available for blood flow in the presence of the elongated device; and
- determining and outputting the value of the pulse-wave-velocity using the second measurement data and the geometry factor.

The method of the third aspect of the invention shares the advantages of the device of the first aspect or of any of its embodiments.

A particular embodiment of the method of the third aspect further comprises:

- determining, using the first measurement data, a first extension value indicative of an average radius of the region of the blood vessel;
- receiving a second extension value indicative of an average radius of a predetermined longitudinal section of the inserted elongated device;
- determining the geometry factor using the first extension value and the second extension value as

$$\sqrt{1 - \frac{r_2^2}{r_1^2}}$$

, wherein $r_1$ is the first average radius value and $r_2$ is the second average radius value; and

- determining the value of the pulse-wave-velocity by dividing the measured transmission velocity amount by the geometry factor.

Yet another embodiment of the method of the third aspect further comprises:

- receiving a vessel-wall thickness value indicative of a thickness of the wall of the blood vessel,
- receiving a density value, indicative of the density of the blood circulating inside the blood vessel; and
- determining a value of an elastic modulus of the vessel using the value of the pulse-wave-velocity, the vessel-wall thickness value and the blood density value in accordance with the calculation rule

$$E = \frac{PWV^2}{h} \cdot 2r_1 \cdot \rho \,,$$

wherein

$E$ is the value of the elastic modulus of the vessel,
$PWV$ is the value of the pulse-wave velocity,
$h$ is the vessel thickness value,
$r_1$ is the average radius value of the vessel, and
$\rho$ is the blood density value.

According to a fourth aspect of the present invention a method for controlling operation of a system for determining a value of a pulse-wave velocity in a region of a blood vessel under measurement is described. The method comprises

- providing first measurement data indicative of vessel geometry parameters of the blood vessel;
- providing the second measurement data representing a measured transmission velocity amount of pressure or flow waves in the region of the vessel; and
- performing a method in accordance with the third aspect of the present invention.

The method of the fourth aspect thus shares the advantages of the system of the second aspect and of any of its embodiments. The method steps of providing the first measurement data and providing the second measurement data can be carried out in any order or simultaneously.

According to a fifth aspect of the present invention, a computer program is disclosed which comprises instructions which, when the program is executed by a computer, cause the computer to carry out the method of the third or the fourth aspects of the invention.

The functions of various units of the device for determining a value of a pulse-wave velocity of a vessel, such as that of the first and second measurement data input units receiving the first and second measurement data, the pulse-wave velocity determination unit and the elastic-modulus determination unit, can be carried out by a single processor or multiple processors of a computer.

It shall be understood that the device, the system, the method for operating a device, the method for operating a system, and the computer program, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows a block diagram of an embodiment of a device for determining a value of a pulse-wave velocity,
Fig. 2 shows a block diagram of another embodiment of a device for determining a value of a pulse-wave velocity,
Fig. 3 shows a block diagram of an embodiment of a system for determining a value of a pulse-wave velocity,
Fig. 4a shows a schematic diagram of a transmission-velocity determining device in an inserted state inside a blood vessel,
Fig. 4b shows a schematic cross-sectional view of the transmission-velocity determining device in the inserted state inside the blood vessel,
Fig. 5a shows a flow diagram of an embodiment of a method for operating a device for determining a value of a pulse-wave velocity,
Fig. 5b shows a flow diagram of another embodiment of a method for operating a device for determining a value of a pulse-wave velocity,
Fig. 5c shows a flow diagram of another embodiment of a method for operating a device for determining a value of a pulse-wave velocity, and

Fig. 6 shows a flow diagram of an embodiment of a method for operating a system for determining a value of a pulse-wave velocity.

## DETAILED DESCRIPTION OF EMBODIMENTS

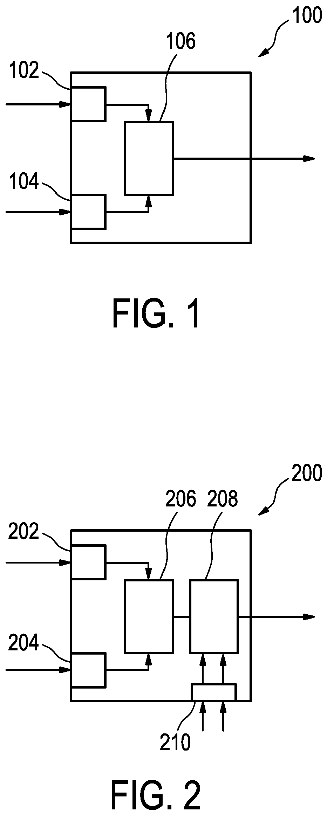

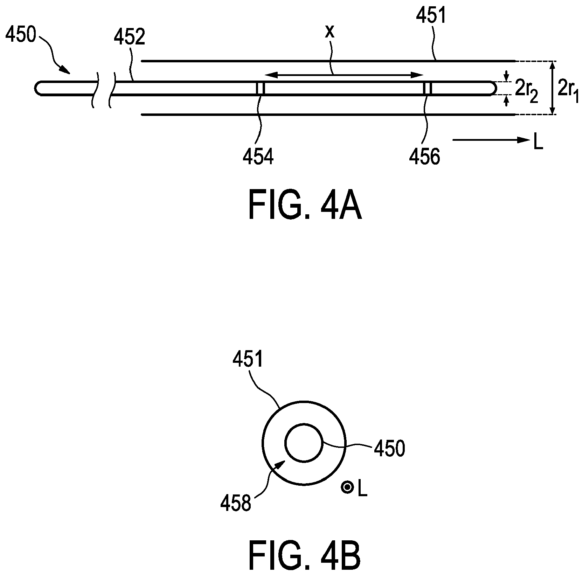

Fig. 1 shows a block diagram representing a device 100 for determining a value of a pulse-wave velocity in a region of a blood vessel under measurement. The description of the device will be further done with reference to Figs. 4a and 4b which show a schematic representation of an elongated device, in particular a transmission-velocity determining device 450 in an inserted state inside a blood vessel 451. The transmission-velocity determining device 450 comprises two pulse wave sensors 454 and 456 arranged at a distance $x$ from each other. Fig. 4a shows a sectional view along a longitudinal direction L, whereas Fig. 4b shows a cross-sectional view thereof. The device 100 comprises a first measurement data input unit 102 that is configured to receive first measurement data axindicative of vessel geometry parameters of the blood vessel 451. An example of such vessel geometry parameters is the value of a radius $r$ of the blood vessel in dependence on a position along the longitudinal direction. A further example is an average value of the radius at a predetermined region of the blood vessel, particularly that region of the blood vessel under measurement. In the exemplary case depicted in Fig 4a and 4b, the average value of the radius of the blood vessel is $r_1$. The device further comprises a second measurement data input unit 104 configured to receive second measurement data representing a measured transmission velocity amount of pressure or flow waves in the region of the blood vessel in presence of an elongate device such as the transmission-velocity determining device 450, which has predetermined spatial extension values, such as, for example, a known radius value $r_2$ of a predetermined section of the transmission-velocity determining device 450 or the distance $x$ separating the pulse wave measurement sensors 454 and 456.

The device 100 further comprises a pulse-wave velocity determination unit 106, which is configured to determine, using the vessel-geometry data and the spatial extension values of the inserted elongated device, a geometry factor dependent on a cross-sectional area of the blood vessel that remains available for blood flow in the presence of the elongated device.

The Navier-Stokes continuity equation translates the conservation of flow over a domain with its varying volume in time. By neglecting the variation of the domain shape in axial direction, thus for a vessel of o cylindrical cross section, the stiffness or compliance of blood vessel depends on the elastic modulus E thereof, resulting in the Moens-Korteweg equation, which models the relationship between pulse-wave velocity and the elastic modulus of the vessel wall,

$$PWV = \sqrt{\frac{Eh}{2r_1\rho}} + U$$

wherein $r_1$ represents the radius of the vessel, $h$ represents the thickness of the vessel wall, $\rho$ represents the density of the blood flowing through the vessel and $U$ is the average blood flow velocity. However, this equation assumes a straight axisymmetrical cylinder with a constant circular cross section and uniform physical properties. In reality, the transmission-velocity determining device 450, or of any other inserted elongated device, in the blood vessel 451 reduces the cross-sectional area 458 that is available for blood flow and influences the measured pulse-wave velocity $PWV$. The resulting artefacts have a similar magnitude as the accuracy required for the vessel compliance estimation. Therefore, those artefacts must be taken into account to increase the significance of the measured pulse-wave velocity. A catheter in the vessel can be approximated with a cylinder having annular cross section, with the inner radius $r_2$ that of the catheter and the outer radius $r_1$ that of the vessel. Accordingly,

$$PWV_m = \sqrt{\frac{Eh(1 - r_2^2/r_1^2)}{2r_1\rho}} + U$$

Thus, the device 100 is configured to determine and output the value of the pulse-wave velocity using the second measurement data, i.e. the measured $PWV_m$, and the geometry factor. In fact the measured $PWV_m$ has to be compensated to approximate the PWV in the case the flow in the vessel would not have been obstructed by the catheter or other measurement device.

An exemplary device for determining a value of a pulse-wave velocity comprises a pulse-wave velocity determination unit that is configured to determine, using the first measurement data (e.g. radiological angiography, magnetic resonance angiography, ultrasound), a first extension value indicative of an average radius of the region of the blood vessel $r_1$, to receive a second extension value indicative of an average radius of a predetermined longitudinal section of the inserted elongated device $r_2$ (by user input, from database, or imaging techniques based on radiology, ultrasound or magnetic resonance) and to determine the geometry factor using the first extension value and the second extension value. A significant expression for the geometry factor has been obtained by applying the Navier-Stokes continuity equation for a tube having an annular cross section. This leads to an expression of the pulse-wave velocity that depends on the blood flow velocity. However, if the absolute value of the blood flow velocity is unknown, it can be neglected without a severe impact on the significance of the value on the pulse-wave velocity obtained. The geometry factor

$G_F$ is in this case

$$G_F = \sqrt{1 - \frac{r_2^2}{r_1^2}}$$

Simulations performed using a computational fluid dynamics (CFD) model using an idealized geometry of a cylinder with and without a tube inserted inside have been used to simulate velocity and pressure fields inside a blood vessel. Vessel wall deformation has also been modelled using a fluid structure interaction (FSI) method. A pressure at an interface with a solid is transmitted as a load on the solid domain. This causes solid displacements which are solved with a linear elastic model, where a deformation or a strain is proportional to a local stress, depending on a stiffness of the material. The equation $\sigma = E \times \varepsilon$ depicts in a simplified way this linear relationship, where $\sigma$ is the load, $\varepsilon$ is the deformation, and $E$ is the Young modulus or elastic modulus quantifying the stiffness of the isotropic material. Since FSI requires a lot of computational power, an axisymmetric approach is chosen for modelling. The blood vessel is modelled as a wedge with a symmetry condition at both side planes. The resulting pulse-wave velocities from the simulation are compared with the pulse-velocities obtained using the geometry factor $G_F$ over a range of parameters and confirms the decrease of the PWV when the diameter of the catheter increases.

Fig. 2 shown a block diagram of another embodiment of a device 200 for determining a value of a pulse-wave velocity in a blood vessel. The following discussion will focus on those features distinguishing the device 100 from the device 200. Features shared by both devices will be referred to using the same numerals except for the first digit, which is "1" for the features of the device 100 of Fig. 1 and "2" for the features of the device 200 of Fig. 2.

The device 200 also comprises an elastic-modulus determination unit 208 that is configured to receive, from the pulse-wave velocity determination unit 206, the value of the pulse-wave velocity PWV and the average radius of the region of the blood vessel $r_1$. It is also configured to receive, via a data input 210, a vessel-wall thickness value indicative of a thickness h of the wall of the blood vessel, and a blood density value, indicative of the density $\rho$ of the blood circulating inside the blood vessel. The elastic-modulus determination unit is also configured to determine a value of an elastic modulus $E$ of the blood vessel using the value of the pulse-wave velocity PWV, the vessel-wall thickness value h and the blood density value $\rho$ by determining

$$E = \frac{PWV^2}{h} \cdot 2r_1 \cdot \rho$$

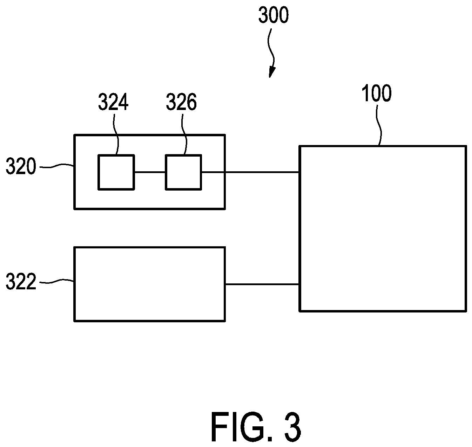

Fig. 3 shows a block diagram representing an embodiment of a system 300 for determining a value of a pulse-wave velocity in a region of a blood vessel under measurement. The system 300 comprises a device 100 for determining a value of a pulse-wave velocity in a region of a vessel. Alternatively, other systems comprise a device 200 that includes an elastic-modulus determination unit. The system 300 further comprises an imaging unit 320 for providing the first measurement data indicative of vessel geometry parameters of the blood vessel, the imagining unit connected to the first measurement data input unit of the device and a transmission-velocity measurement device 322 for providing the second measurement data representing a measured transmission velocity amount of pressure or flow waves in the region of the vessel, the transmission-velocity measurement device connected to the second measurement data input unit of the device.

In system 300, the imaging unit 320 comprises an image-acquiring device 324 configured to provide image data indicative of the region of the blood vessel and a segmentation unit 326 configured to receive the image data and to perform a segmentation algorithm for determining and providing the vessel-geometry data indicative of the vessel geometry to the device 100.

Alternative systems for determining a value of a pulse-wave velocity comprise different image-acquiring devices, such as, but not limited to image-acquiring devices comprising an ultrasound probe, a computer tomography scanner or a magnetic resonance imaging scanner.

The transmission-velocity measurement device 322 is in some particular systems a transmission-velocity measurement device 450 such as that described with reference to Fig. 4a and 4b. The transmission-velocity measurement device 450 comprises two pulse wave sensors 454 and 456 arranged at respective predetermined positions along a longitudinal direction of the catheter. A distance separating both pulse wave sensors is given by x in Fig. 4a. Other transmission-velocity measurement devices comprise more than two pulse wave sensors. The pulse wave sensors are in a particular transmission-velocity measurement device, pressure sensors, flow sensors, imaging sensors or a combination thereof.

The transmission-velocity measurement device is configured to provide a value of a measured transmission velocity amount of pressure or flow waves in the region of the blood vessel. A simple way of obtaining this value is by dividing the value of the distance x separating both pulse wave sensors by a time difference value $\Delta t$ indicative of a time difference at which a predetermined pressure or flow wave has been detected at each pulse wave sensor. Thus,

$$PWV_{measured} = \frac{x}{\Delta t}$$

The significance of this value, which is still influenced by the presence of the transmission-velocity measurement device inside the vessel, is increased at the device 100 by determining a geometry factor and providing a value of the pulse-wave velocity based thereupon.

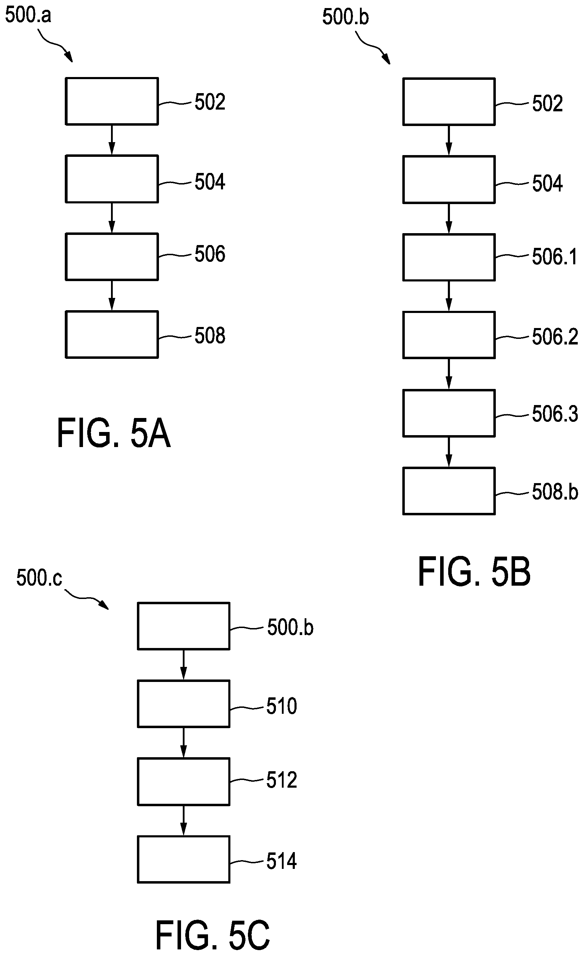

Fig. 5a shows a flow diagram of an embodiment of a method 500.a for operating a device for determining a value of a pulse-wave velocity in a region of a blood vessel under measurement. The method comprises, in a step 502, receiving at a first measurement data input unit, first measurement data indicative of vessel geometry parameters of the blood vessel. It also comprises, in a step 504, receiving, at a second measurement data input unit, second measurement data representing a measured transmission velocity amount of pressure or flow waves in the region of the vessel in presence of an inserted elongated device having predetermined spatial extension values. Steps 502 and 504 can be carried out in any order. The method 500.a also comprises, in a step 506, determining, using the vessel-geometry data and the spatial extension values of the elongated device, a geometry factor dependent on a cross-sectional area of the blood vessel that remains available for blood flow in the presence of the elongated device. Finally, in a step 508, the method 500.a comprises determining and outputting 508 the value of the pulse-wave velocity using the second measurement data and the geometry factor.

Fig. 5b shows a flow diagram of another embodiment of a method 500.b for operating a device for determining a value of a pulse-wave velocity. The method 500.b also comprises, in a step 502, receiving at a first measurement data input unit, first measurement data indicative of vessel geometry parameters of the blood vessel, and in a step 504, receiving, at a second measurement data input unit, second measurement data representing a measured transmission velocity amount of pressure or flow waves in the region of the vessel in presence of an elongated device having predetermined spatial extension values. As in the case of method 500.a of Fig. 5a, steps 502 and 504 can be carried out in any order or simultaneously. Method 500.b then comprises, in a step 506.1, determining, using the first measurement data, a first extension value indicative of an average radius of the region of the blood vessel, in a step 506.2, receiving a second extension value indicative of an average radius of a predetermined longitudinal section of the elongated device and in a step 506.3, determining the geometry factor using the first extension value and the second extension value as $\sqrt{1 - \dfrac{r_2^2}{r_1^2}}$ wherein $r_1$ is the first average radius value and $r_2$ is the second average radius value. Finally, the method 500.b comprises, in a step 508.b, determining the value of the pulse-wave velocity by dividing the measured transmission velocity amount by the geometry factor.

Fig. 5c shows a flow diagram of another embodiment of a method 500.c for operating a device for determining a value of a pulse-wave velocity. The method includes performing the steps of method 500.b to determine the value of the pulse-wave velocity. The method 500.c then includes, in a step 510, receiving a vessel-wall thickness value indicative of a thickness of the wall of the blood vessel, in a step 512, receiving a density value, indicative of the density of the blood circulating inside the blood vessel and, in a step 514, determining a value of an elastic modulus E of the vessel using the value of the pulse-wave-velocity PWV, the vessel-wall thickness value $h$ and the blood density value $\rho$ in accordance with the calculation rule

$$E = \frac{PWV^2}{h} \cdot 2r_1 \cdot \rho \; ;$$

wherein $r_1$ is the average radius value of the vessel.

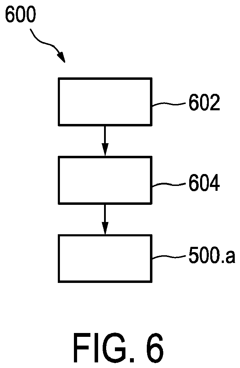

Fig. 6 shows a flow diagram of an embodiment of a method 600 for operating a system for determining a value of a pulse-wave velocity in a region of a blood vessel under measurement. The method comprises, in a step 602, providing first measurement data indicative of vessel geometry parameters of the blood vessel. In a step 604, the method comprises providing second measurement data representing a measured transmission velocity amount of pressure or flow waves in the region of the vessel. Steps 602 and 604 can be performed in any order or simultaneously. Finally, the method comprises performing the steps of method 500.a, described with reference to Fig. 5a. Alternatively, in other embodiments of the method 600 the steps of methods 500.b and 500.c are performed instead of the steps of method 500.a.

In summary, the present invention is directed to a device for determining a value of a pulse-wave velocity in a blood vessel under measurement, that comprises a first measurement data input unit configured to receive data indicative of vessel geometry parameters of the blood vessel, a second measurement data input unit configured to receive data representing a measured transmission velocity amount of pressure waves in the blood vessel in presence of an elongated device having predetermined spatial extension values and a pulse-wave velocity determination unit, which is configured to determine, using the vessel-geometry data and the spatial extension values of the elongated device, a geometry factor dependent on a cross-sectional area of the blood vessel that remains available for blood flow in the presence of the elongated device and to determine and output the value of the pulse-wave-velocity using the second measurement data and the geometry factor.

Claims

1. A device (100) for determining a value of a pulse-wave velocity in a region of a blood vessel under measurement, the device comprising:

- a first measurement data input unit (102) configured to receive first measurement data indicative of vessel geometry parameters of the blood vessel;
- a second measurement data input unit (104) configured to receive second measurement data representing a measured transmission velocity amount of pressure or flow waves in the region of the blood vessel in presence of an inserted elongated device having predetermined spatial extension values; and
- a pulse-wave velocity determination unit (106), which is configured

- to determine, using the vessel-geometry data and the spatial extension values of the inserted elongated device, a geometry factor dependent on a cross-sectional area of the blood vessel that remains available for blood flow in the presence of the inserted device; and
to determine and output the value of the pulse-wave velocity using the second measurement data and the geometry factor.

2. The device of claim 1, wherein the pulse-wave velocity determination unit is configured

- to determine, using the first measurement data, a first extension value indicative of an average radius of the region of the blood vessel;
- to receive a second extension value indicative of an average radius of a predetermined longitudinal section of the inserted elongated device; and
- to determine the geometry factor using the first extension value and the second extension value.

3. The device of claim 2, wherein the pulse-wave velocity determination unit is configured to determine the geometry factor as $\sqrt{1 - \dfrac{r_2^2}{r_1^2}}$ , wherein

$r_1$ is the first extension value,
$r_2$ is the second extension value,

and wherein the value of the pulse-wave velocity is determined by dividing the measured transmission velocity amount by the geometry factor.

4. The device of claim 2, further comprising an elastic-modulus determination unit (208) configured

- to receive, from the pulse-wave velocity determination unit, the value of the pulse-wave velocity and the average radius of the region of the blood vessel;
- to receive, via a data input (210), a vessel-wall thickness value indicative of a thickness of the wall of the blood vessel, and a blood density value, indicative of the density of the blood circulating inside the blood vessel; and
- to determine a value of an elastic modulus of the blood vessel using the value of the pulse-wave velocity, the vessel-wall thickness value and the blood density value by determining:

$$E = \frac{PWV^2}{h} \cdot 2r_1 \cdot \rho \, ,$$

wherein

$E$ is the value of the elastic modulus of the blood vessel,
$PWV$ is the value of the pulse-wave velocity,
$h$ is the vessel thickness value of the blood vessel,
$r_1$ is the average radius of the region of the blood vessel, and
$\rho$ is the blood density value.

5. A system (300) for determining a value of a pulse-wave velocity in a region of a blood vessel under measurement, the system comprising:

- a device for determining a value of a pulse-wave velocity in a region of a vessel under measurement in accordance with claim 1;
- an imaging unit (320) for providing the first measurement data indicative of vessel geometry parameters of the blood vessel, the imaging unit connected to the first measurement data input unit of the device; and
- a transmission-velocity measurement device (322) for providing the second measurement data representing a measured transmission velocity amount of pressure or flow waves in the region of the vessel, the transmission-velocity measurement device connected to the second measurement data input unit of the device.

6. The system of claim 5, wherein the imaging unit comprises:

- an image-acquiring device (324) configured to provide image data indicative of the region of the blood vessel; and
- a segmentation unit (326) configured

- to receive the image data;

- to perform a segmentation algorithm for determining and providing the vessel-geometry data indicative of the vessel geometry.

7. The system of claim 6, wherein the image-acquiring device comprises an ultrasound probe or a computed tomography scanner or a magnetic resonance imaging scanner or an angiography-imaging device.

8. The system of claim 6, wherein transmission-velocity measurement device comprises a catheter or a guidewire having predetermined spatial extension values, the catheter or the guidewire including at least two pulse wave sensors arranged at respective predetermined positions along a longitudinal direction of the catheter or the guidewire.

9. The system of claim 6, wherein the transmission velocity measurement device comprises two or more catheters or guidewires having predetermined spatial extension values, each catheter or guidewire including at least one pulse wave sensors arranged at respective predetermined positions along a longitudinal direction of the respective catheter or guidewire.

10. The system of claims 8 or 9, wherein the pulse wave sensors are pressure sensors or flow sensors or imaging sensors or a combination thereof.

11. A method for operating a device (500.a) for determining a value of a pulse-wave velocity in a region of a blood vessel under measurement, the method comprising:

- receiving (502), at a first measurement data input unit, first measurement data indicative of vessel geometry parameters of the blood vessel;
- receiving (504), at a second measurement data input unit, second measurement data representing a measured transmission velocity amount of pressure or flow waves in the region of the vessel in presence of an inserted elongated device having predetermined spatial extension values;
- determining (506), using the vessel-geometry data and the spatial extension values of the elongated device, a geometry factor dependent on a cross-sectional area of the blood vessel that remains available for blood flow in the presence of the elongated device; and
- determining and outputting (508) the value of the pulse-wave-velocity using the second measurement data and the geometry factor.

12. The method of claim 11, further comprising:

- determining (506.1), using the first measurement data, a first extension value indicative of an average radius of the region of the blood vessel;
- receiving (506.2) a second extension value indicative of an average radius of a predetermined longitudinal section of the elongated device;
- determining the geometry factor (506.3) using the first extension value and the second extension value as $\sqrt{1-\dfrac{r_2^2}{r_1^2}}$, wherein $r_1$ is the first average radius value and $r_2$ is the second average radius value; and
- determining (508.b) the value of the pulse-wave velocity by dividing the measured transmission velocity amount by the geometry factor.

13. The method of claim 12, further comprising:

- receiving (510) a vessel-wall thickness value indicative of a thickness of the wall of the blood vessel;
- receiving (512) a density value, indicative of the density of the blood inside the blood vessel; and
- determining (514) a value of an elastic modulus of the vessel using the value of the pulse-wave velocity, the vessel-wall thickness value and the blood density value in accordance with the calculation rule

$$E = \frac{PWV^2}{h} \cdot 2r_1 \cdot \rho \,,$$

wherein

$E$ is the value of the elastic modulus of the vessel,
$PWV$ is the value of the pulse-wave velocity,
$h$ is the vessel thickness value,
$r_1$ is the average radius value of the vessel, and
$\rho$ is the blood density value.

14. A method (600) for operating a system for determining a value of a pulse-wave velocity in a region of a blood vessel under measurement, the method comprising:

- providing (602) first measurement data indicative of vessel geometry parameters of the blood vessel;
- providing (604) second measurement data representing a measured transmission velocity amount of pressure or flow waves in the region of the vessel; and
- performing a method (500) in accordance with claim 11.

15. A computer program comprising instructions which, when the program is executed by a computer, cause the computer to carry out the method of claim 11.

FIG. 1

FIG. 2

FIG. 3

450　452　　　　　　　　x　　　　　　451

$2r_2$　$2r_1$

454　　　　　456　　　　　L

## FIG. 4A

451

450

458　　⊙L

## FIG. 4B

500.a

502

504

506

508

FIG. 5A

500.b

502

504

506.1

506.2

506.3

508.b

FIG. 5B

500.c

500.b

510

512

514

FIG. 5C

600

602

604

500.a

FIG. 6

Europäisches
Patentamt

European
Patent Office

Office européen
des brevets

## EUROPEAN SEARCH REPORT

Application Number

EP 18 18 2826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/313479 A1 (STIGALL JEREMY [US] ET AL) 5 November 2015 (2015-11-05) * paragraph [0008] - paragraph [0012] * * paragraph [0037] - paragraph [0048] * ----- | 1-15 | INV. A61B5/00 A61B5/021 A61B5/027 |
| A | WO 2012/155040 A1 (ACIST MEDICAL SYS INC [US]; SUCHECKI TODD M [US]; EVANS ALAN K [US]) 15 November 2012 (2012-11-15) * abstract * * page 20, line 16 - page 22, line 6 * ----- | 1-15 | |
| A | WO 2017/198813 A1 (KONINKLIJKE PHILIPS NV [NL]) 23 November 2017 (2017-11-23) * abstract * * page 12, line 29 - line 34 * ----- | 1-15 | |
| A | US 4 425 920 A (BOURLAND JOE D [US] ET AL) 17 January 1984 (1984-01-17) * column 4, line 1 - line 27 * ----- | 4 | |

TECHNICAL FIELDS
SEARCHED     (IPC)

A61B

The present search report has been drawn up for all claims

| Place of search | Date of completion of the search | Examiner |
|---|---|---|
| The Hague | 9 January 2019 | Van Dop, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 2826

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2019

| Patent document cited in search report | | Publication date | Patent family member(s) | | Publication date |
|---|---|---|---|---|---|
| US 2015313479 | A1 | 05-11-2015 | CN | 106456073 A | 22-02-2017 |
| | | | EP | 3136966 A1 | 08-03-2017 |
| | | | JP | 2017518151 A | 06-07-2017 |
| | | | US | 2015313479 A1 | 05-11-2015 |
| | | | WO | 2015168502 A1 | 05-11-2015 |
| WO 2012155040 | A1 | 15-11-2012 | CN | 103796578 A | 14-05-2014 |
| | | | EP | 2706908 A1 | 19-03-2014 |
| | | | JP | 6059207 B2 | 11-01-2017 |
| | | | JP | 2014517752 A | 24-07-2014 |
| | | | US | 2013131523 A1 | 23-05-2013 |
| | | | US | 2016106373 A1 | 21-04-2016 |
| | | | US | 2017360376 A1 | 21-12-2017 |
| | | | WO | 2012155040 A1 | 15-11-2012 |
| WO 2017198813 | A1 | 23-11-2017 | CN | 109152540 A | 04-01-2019 |
| | | | WO | 2017198813 A1 | 23-11-2017 |
| US 4425920 | A | 17-01-1984 | NONE | | |

EPO FORM P0459

For more details about this annex : see Official Journal of the European Patent Office, No. 12/82

**EP 3 593 706 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100113949 A1